# EUROPEAN PATENT SPECIFICATION

(11) **EP 4 182 928 B1**
(45) Date of publication and mention of the grant of the patent: **04.09.2024**
(21) Application number: 21742134.6
(22) Date of filing: 12.07.2021
(51) Int. Cl.: G16B 30/00, G16B 40/20

(54) **METHOD, SYSTEM AND COMPUTER PROGRAM PRODUCT FOR DETERMINING PRESENTATION LIKELIHOODS OF NEOANTIGENS**
VERFAHREN, SYSTEM UND COMPUTERPROGRAMMPRODUKT ZUR BESTIMMUNG VON PRÄSENTATIONSWAHRSCHEINLICHKEITEN VON NEOANTIGENEN
PROCÉDÉ, SYSTÈME ET PRODUIT DE PROGRAMME INFORMATIQUE PERMETTANT DE DÉTERMINER LES PROBABILITÉS DE PRÉSENTATION DE NÉO-ANTIGÈNES

(30) Priority: 14.07.2020 EP 20185779
(43) Date of publication of application: 24.05.2023
(73) Proprietor: MYNEO NV, 9000 Gent (BE)
(72) Inventor: FANT, Bruno, 9000 Gent (BE); BOGAERT, Cedric, 9000 Gent (BE); ADELL MILL, Nil, 9000 Gent (BE)
(74) Representative: Brantsandpatents bv
(86) International application number: PCT/EP2021/069341
(87) International publication number: WO 2022/013154

(56) References cited:
- US-A1- 2020 105 377
- BRENDAN BULIK-SULLIVAN ET AL: "Deep learning using tumor HLA peptide mass spectrometry datasets improves neoantigen identification", NATURE BIOTECHNOLOGY, vol. 37, no. 1, 17 December 2018 (2018-12-17), us, pages 55 - 63, XP055644930, ISSN: 1087-0156, DOI: 10.1038/nbt.4313

## Description

### FIELD OF THE INVENTION

The invention pertains to a computer-implemented method, computer system and computer program product for determining presentation likelihoods of neoantigens.

### BACKGROUND

In addition to normal epitopes, the surfaces of cancer cells are likely to present neoantigens, derived from aberrant genomic events, and recognizable by T-cells.

Neoantigens are newly formed antigens that have not been previously recognized by the immune system. In recent years, targeting these neoantigens has shown to be a very promising avenue of personalized medicine.

New technological developments have allowed for the increased availability of mass spectrometry-derived lists of peptides that are actually bound to major histocompatibility complex (MHC) molecules at the cell surface. These lists are called "ligandomes". Current state of the art neoantigen discovery methods start by generating a list of all potential neoantigens produced by cancer cells and rely on *in silico* prediction algorithms in order to extract the epitopes that are most likely to be present at the surface of these cells, potentially eliciting an immune reaction.

WO 2017 106 638 describes a method for identifying one or more neoantigens from a tumor cell of a subject that are likely to be presented on the tumor cell surface. Moreover, the document discloses systems and methods for obtaining high quality sequencing data from a tumor and for identifying somatic changes in polymorphic genome data. Finally, WO '638 describes unique cancer vaccines.

US 2019 0 311 781 describes a method for identifying peptides that contain features associated with successful cellular processing, transportation and MHC presentation, through the use of a machine learning algorithm or statistical inference model. US 2018 0 085 447 describes a method for identifying immunogenic mutant peptides having therapeutic utility as cancer vaccines. More specifically, a method for identifying T-cell activating neoepitopes from all genetically altered proteins. These mutated proteins contribute to neoepitopes after they are degraded by means of proteolysis within antigen presenting cells.

EP 3 256 853 describes a method for predicting T-cell epitopes useful for vaccination. In particular, the document relates to methods for predicting whether modifications in peptides or polypeptides such as tumor-associated neoantigens are immunogenic and, in particular, useful for vaccination, or for predicting which of such modifications are most immunogenic and, in particular, most useful for vaccination.

US 2020/105377 A1 discloses methods for identifying neoantigens from a tumor of a subject that are likely to be presented on the cell surface of the tumor and/or are likely to be immunogenic. In one example, the method comprises the steps of: obtaining at least one of exome, transcriptome or whole genome tumor nucleotide sequencing data from the tumor cell of the subject, wherein the tumor nucleotide sequencing data is used to obtain data representing peptide sequences of each of a set of neoantigens, and wherein the peptide sequence of each neoantigen comprises at least one alteration that makes it distinct from the corresponding wild-type, parental peptide sequence; inputting the peptide sequence of each neoantigen into one or more presentation models to generate a set of numerical likelihoods that each of the neoantigens is presented by one or more MHC alleles on the tumor cell surface of the tumor cell of the subject or cells present in the tumor, the set of numerical likelihoods having been identified at least based on received mass spectrometry data; and selecting a subset of the set of neoantigens based on the set of numerical likelihoods to generate a set of selected neoantigens.

Several further tools and approaches are available which address the same issue, such as NetMHCpan or MHCflurry. These approaches use methods that predict a peptide binding affinity to a given HLA allele. Other approaches, such as EDGE or MARIA, also output learning-based presentation probabilities, but do not account for the HLA sequence and encode the HLA type as a categorical variable.

Furthermore, initial prediction methods use binding affinity of candidate neoantigens to the MHC as an indicator for likelihood of presence at the cell surface. These approaches, however, fail to model the entirety of the cell surface presentation process, and therefore suffer from low positive predictive values. Moreover, these approaches are not able to predict the presentation likelihoods of neoepitopes for HLA molecules that are not included in the training of the model.

The invention aims to provide a solution to at least some of the disadvantages discussed hereabove, as well as improvements over the state-of-the-art techniques.

### SUMMARY OF THE INVENTION

In a first aspect, the invention pertains to a computer-implemented method for determining presentation likelihoods of a set of neoantigens by a tumour cell of a tumour of a subject according to claim 1.

I a second aspect, the invention pertains to a computer system for determining presentation likelihoods of a set of neoantigens by a tumour cell of a tumour of a subject according to claim 12.

In a third aspect, the invention pertains to a computer program product for determining presentation likelihoods of a set of neoantigens by a tumour cell of a tumour of a subject according to claim 13.

In fourth aspect the invention pertains to a use for determining a treatment for the subject according to claim 14.

The object of the invention is predicting likelihoods of presentation at a cancer cell surface of a variable-length neoepitope given a set of HLA alleles expressed by said cell. To this end a deep learning model is used.

The invention is advantageous as presentation likelihoods of neoepitopes to any HLA allele can be predicted even if the model has not been trained on the HLA allele.

Preferred embodiments of the invention are discussed in claims 2 to 12, as well as throughout the description and examples.

### FIGURES

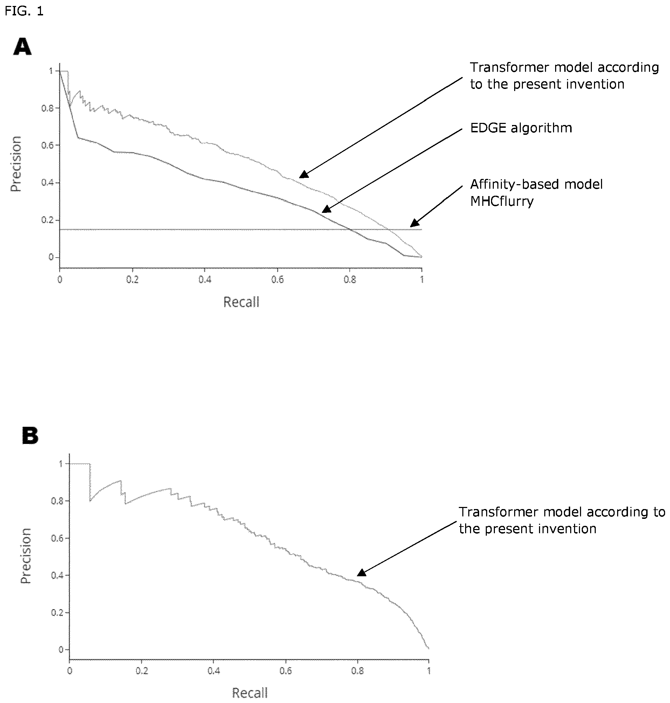

**Figure 1** shows precision-recall curves obtained as a result of testing a model according to the present invention on test datasets. **Figure 1A** shows a comparison in performance of a model according to the present invention and prior art algorithms EDGE algorithm and MHCflurry algorithm when tested on the same test dataset. **Figure 1B** shows the predictive power of a model according to the present invention when tested on a new dataset.

### DETAILED DESCRIPTION OF THE INVENTION

The invention pertains, in a first aspect, to a computer-implemented method for determining presentation likelihoods of a set of neoantigens. In a second and third aspect, the invention pertains to a computer system and a computer program product. In a fourth aspect, the invention pertains use of any of the method, system or product for determining a treatment for the subject. In what follows, the invention will be described in detail, preferred embodiments are discussed, and the invention will be illustrated by means of non-limitative examples.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

As used herein, the following terms have the following meanings:
"A," "an," and "the" as used herein refers to both singular and plural referents unless the context clearly dictates otherwise. By way of example, "a compartment" refers to one or more than one compartment.

"Comprise," "comprising," and "comprises" and "comprised of" as used herein are synonymous with "include," "including," "includes" or "contain," "containing," "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. All percentages are to be understood as percentage by weight unless otherwise defined or unless a different meaning is obvious to the person skilled in the art from its use and in the context wherein it is used. The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se*, by means of further exemplification, the term encompasses *inter alia* a reference to any one of said members, or to any two or more of said members, such as, *e*.*g*., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

In a first aspect, the invention pertains to a computer-implemented method for determining presentation likelihoods of a set of neoantigens by a tumour cell of a tumour of a subject. The method preferably comprising the step of obtaining at least one of exome or whole genome nucleotide sequencing data and transcriptome nucleotide sequencing data from tumour cells associated to said tumour and normal cells of the subject. The method preferably further comprising the step of obtaining a set aberrant genomic events associated to said tumour by comparing the exome and/or whole genome nucleotide sequencing data and the transcriptome nucleotide sequencing data from the tumour cells to the exome and/or whole genome nucleotide sequencing data and the transcriptome nucleotide sequencing data from the normal cells. The method preferably further comprising the step of obtaining data representing peptide sequences of each of a set of neoantigens identified based at least in part on said set of aberrant events, wherein the peptide sequence of each neoantigen comprises at least one alteration which makes it distinct from a corresponding wild-type peptide sequence identified from the normal cells of the subject. The method preferably further comprising the step of obtaining data representing a peptide sequence of an HLA based on the tumour exome and/or whole genome nucleotide sequencing data and the transcriptome nucleotide sequencing data from the tumour cells. The method preferably further comprising the step of training a deep learning model on a training data set comprising a positive data set, wherein the positive data set comprises a plurality of input-output pairs, wherein each pair comprises an entry of an epitope sequence as input, said epitope sequence being identified or inferred from a surface bound or secreted HLA/peptide complex encoded by a corresponding HLA allele expressed by a training cell, wherein each pair further comprises an entry of a peptide sequence of an alpha-chain encoded by the corresponding HLA allele as output. The method preferably further comprising the step of determining a presentation likelihood for each of the set of neoantigens for the peptide sequence of the HLA by means of the trained model.

In a second aspect, the invention pertains to a computer system for determining presentation likelihoods of a set of neoantigens by a tumour cell of a tumour of a subject. The computer system configured for performing the computer-implemented method according to the first aspect of the invention.

In a third aspect, the invention pertains to a computer program product for determining presentation likelihoods of a set of neoantigens by a tumour cell of a tumour of a subject. The computer program product comprising instructions which, when the computer program product is executed by a computer, cause the computer to carry out the method according to the first aspect of the invention.

In fourth aspect the invention pertains to a use of the method according to the first aspect of the invention and/or the computer system according to the second aspect of the invention and/or the computer program product according to the third aspect of the invention, for determining a treatment for the subject.

The invention provides a computer-implemented method, a computer system and a computer program product for determining presentation likelihoods of neoantigens by a tumour cell of a tumour of a subject, as well a use of any of any of the method, system or product for determining a treatment for the subject. A person having ordinary skill in the art will appreciate that the method is implemented in the computer program product and executed using the computer system. It is also clear for a person having ordinary skill in the art that presentation likelihoods of a set of neoantigens can be used for determining a treatment for the subject. In what follows, the four aspects of the present invention are therefore treated together.

"Subject," as used herein, refers to a term known in the state of the art, that should preferably be understood as a human or animal body, most preferably a human body. As used herein, "animal" preferably refers to vertebrates, more preferably to birds and mammals, even more preferably mammals. "Subject in need thereof," as used herein, should be understood as a subject who will benefit from treatment.

A simple embodiment of the invention preferably provides obtaining at least one of exome or whole genome nucleotide sequencing data and transcriptome nucleotide sequencing data from tumour cells associated to said tumour and normal cells of the subject. A simple embodiment preferably further provides the step of obtaining a set aberrant genomic events associated to said tumour by comparing the exome and/or whole genome nucleotide sequencing data and the transcriptome nucleotide sequencing data from the tumour cells to the exome and/or whole genome nucleotide sequencing data and the transcriptome nucleotide sequencing data from the normal cells. It is clear, that the exome, whole genome nucleotide sequencing data and transcriptome nucleotide sequencing data are each respectively compared to the corresponding nucleotide sequencing data-data type.

"Neoepitope," as used herein, refers to a term known in the state of the art, that should preferably be understood as a class of major histocompatibility complex (MHC) bound peptides that arise from tumour-specific mutations. These peptides represent the antigenic determinants of neoantigens. Neoepitopes are recognized by the immune system as targets for T-cells and can elicit immune responses to cancer.

"Neoantigen," as used herein, refers to a term known in the state of the art, that should preferably be understood as an antigen that has at least one alteration that makes it distinct from the most closely related wild-type antigen, i.e. corresponding wild-type sequence, e.g. via tumour cell mutation, post-translational modification specific to a tumour cell, fusion, transposable elements insertion, alternative splicing event, or any way of alteration known by a person skilled in the art. Furthermore, a neoantigen may or may not include a polypeptide or nucleotide sequence.

Preferably, the set aberrant genomic events comprising one or more of Single-nucleotide polymorphism (SNP), indel mutations, gene fusions, chromosomal rearrangements such as inversion, translocation, duplication, or chronotropisms', transposable element insertions or alternative splicing events. Within the context of this description, the term "indel" is to be understood as a molecular biology term for an insertion or deletion of one or more nucleic acids in the genome of an organism. Furthermore, within the context of this description, the term "SNP" or "single-nucleotide polymorphism" refers to a substitution of a single nucleotide that occurs at a specific position in the genome of an organism.

The present invention may or may not use inputs peptide or neoepitope sequences generated by a neoepitope discovery pipeline, starting from raw sequencing data from a subject, preferably a patient. This raw sequencing data comprises at least tumour DNA, preferably biopsy-generated tumour DNA. Preferably, this raw data further comprises tumour RNA, more preferably biopsy-generated tumour RNA. Preferably, this raw data further comprises normal DNA generated from a sample of the subject, preferably a blood sample. Preferably, this raw data further comprises normal RNA generated from a sample of the subject, preferably a blood sample.

"Sample," as used herein, refers to a term known in the state of the art, that should preferably be understood as a single cell or multiple cells or fragments of cells or an aliquot of body fluid, taken from a subject, by means of, including, venipuncture, excretion, ejaculation, massage, biopsy, needle aspirate, lavage sample, scraping, surgical incision, or intervention or any other means known in the art.

The neoepitope discovery pipeline outputs a list of all genome- and transcriptome-altering events occurring within the tumour. These "aberrant genomic events" comprise novel transposable elements insertion events, novel RNA isoforms, novel gene fusions, novel RNA editing events as well as novel nucleotide-based Post-Translational Modifications events on produced proteins. In addition, it detects single nucleotide polymorphisms (SNPs) and indels (localized insertion or deletion mutations) both on an RNA and DNA level and confronts the results from both analyses to produce a list of high-confidence SNPs and indels.

According to a preferred embodiment, a confidence score is associated to each of said set of aberrant genomic events based at least in part on a number of sequencing reads of the sequencing data supporting each associated aberrant genomic event.

Preferably, the confidence score further based at least in part on a pervasive in the genome of the sequencing data supporting each associated aberrant genomic event. The preferred embodiment, further comprising obtaining a sub-set of aberrant genomic events by comparing the confidence score of each aberrant genomic event of said set of aberrant genomic events to a threshold value, wherein an event is added to said sub-set if the associated confidence score exceeds said threshold value. The set of neoantigens identified based at least in part on said set of aberrant events are, according to the present preferred embodiment, identified based at least in part on said sub-set of aberrant events. Events with a high confidence score display a high number of sequencing reads and are pervasive in the genome and are thus selected for further research. As a consequence, performance is improved.

It should be noted that the invention will not work if the input sequence includes non-canonical amino acids. Within the context of this description, the term "non-canonical amino acids" is to be understood as non-standard or non-coded amino acids, which are not naturally encoded or found in the genetic code of any organism.

A simple embodiment of the invention preferably provides obtaining data that represents a peptide sequence of an HLA based on the tumour exome and/or whole genome nucleotide sequencing data and the transcriptome nucleotide sequencing data from the tumour cells. Thus, HLA makeup of a tumour biopsy is assessed using the same genomic data used for identifying the set of neoantigens. Preferably, the invention provides obtaining data that represents a peptide sequence of each of a set of HLAs based on the tumour exome and/or whole genome nucleotide sequencing data and the transcriptome nucleotide sequencing data from the tumour cells.

"Human leukocyte antigen (HLA)," as used herein, refers to a term known in the state of the art, that should preferably be understood as a gene complex encoding the "major histocompatibility complex (MHC)" proteins in humans. These cell surface proteins are responsible for the regulation of the immune system in humans. HLA genes are highly polymorphic, i.e. having may different alleles, which allows them to fine-tune the adaptive immune system of a subject. Within the context of this description, the term "HLA binding affinity" or "MHC binding affinity" is to be understood as affinity of binding between a specific antigen and a specific MHC allele. Within the context of this description, the term "HLA type" is to be understood as the complement of HLA gene alleles.

A simple embodiment of the invention preferably provides training a deep learning model on a training data set. The training data set preferably comprising a positive data set. The positive data set preferably comprising a plurality of input-output pairs. Each pair preferably comprising an entry of an epitope sequence as input. The epitope sequence preferably being identified or inferred from a surface bound or secreted HLA/peptide complex encoded by a corresponding HLA allele expressed by a training cell. Each pair preferably further comprising an entry of a peptide sequence of an alpha-chain encoded by the corresponding HLA allele as output.

"Training cell," as used herein, should preferably be understood as a cell from which a sample is derived and wherein said sample is used for obtaining the input and output of an input-output pair in the positive data set. The training cell may or may not be a cell obtained from a monoallelic cell line, such as a human cell line, or a cell obtained from a multiallelic tissue, such as a human tissue.

According to a most preferred embodiment, each positive input consists out of the sequence of an epitope consisting of 8 to 15 amino acids, that was shown to be present at the cell surface in a given dataset. Each associated positive output is made of the concatenated amino acid sequence, up to 71 amino acids, of the alpha chains of the HLA allele(s) expressed by the cell in the same dataset.

According to a preferred embodiment, the epitope sequences of the inputs of each input-output pair of the positive data set are obtained by mass spectrometry. In another or further embodiment, the peptide sequence of an alpha-chain encoded by the corresponding HLA allele of the outputs of each input-output pair of the positive data set are obtained by mass spectrometry.

In an embodiment of the invention, positive input-output pairs can be assigned different weights, preferably depending on the frequency of occurrence in the mass spectrometry data used to build the positive training set. The weights modify the impact the pairs have on the training of the deep learning model. A larger weight will lead to a larger adjustment of parameters associated to the deep learning model when training the model with said input-output pair, as is explained further below.

According to a further preferred embodiment, the training data set for training the deep learning model further comprises a negative data set. The negative data set preferably comprising a plurality of input-output pairs. Each pair preferably comprising an entry of a peptide sequence as input. Said peptide sequence preferably being a random sequence of a human proteome. Each pair preferably further comprising a peptide sequence encoded from a random HLA allele as output.

According to a most preferred embodiment, each positive input is a random sequence from the human proteome not present in any ligandome dataset. The inputs are random sequences consisting of 8 to 15 amino acids. Each associated output is a concatenation of the sequence of the alpha chains of a random set of HLA allele(s) present in the positive dataset.

"Proteome," as used herein, refers to a term known in the state of the art, that should preferably be understood as the entire set of proteins that is, or can be, expressed by a genome, cell, tissue, or organism at a certain time. It is the set of expressed proteins in a given type of cell or organism, at a given time, under defined conditions. "Proteomics" is the study of the proteome.

Preferably, a part, preferably a majority, of the input-output pairs of the positive data set, more preferably of both the positive and negative data set, is used for training the deep learning model. Preferably, a part, preferably a minority, of the input-output pairs of the positive data set, more preferably of both the positive and negative data set, is used for validating the trained deep learning model.

A ratio between the number of positive and negative input-output pairs for training the deep learning model may or may not vary. Said ratio is an important parameter of the training of the model.

A ratio between the number of positive and negative input-output pairs for validation the deep learning model may or may not vary. Said ratio is an important parameter of the validation of the model.

According to a preferred embodiment, the positive data set comprises a monoallelic and multiallelic data set. The monoallelic data set preferably comprising input-output pairs obtained from a training cell from a monoallelic cell line. The multiallelic data set preferably comprising input-output pairs obtained from a training cell from a multiallelic tissue. The training cell obtained from a monoallelic cell line preferably being a cell obtained from a monoallelic human cell line. The training cell obtained from a multiallelic tissue preferably being a cell obtained from a human tissue. The multiallelic human tissue may or may not be healthy or cancerous.

"Monoallelic," as used herein, refers to a term known in the state of the art, that should preferably be understood as a situation when only one allele occurs at a site or locus in a population.

"Multiallelic," as used herein, refers to a term known in the state of the art, that should preferably be understood as a situation when many alleles occur. The polymorphism is "multiallelic," also referred to as "polyallelic".

According to a preferred embodiment, training of the deep learning model comprises two or more training cycles. Each training cycle preferably comprising a plurality of training steps. Each training step preferably comprising processing a pair of the plurality of input-output pairs. Preferably, one of said two or more training cycles comprises training the deep learning model on the monoallelic data set. Preferably, one of said two or more training cycles comprises training the deep learning model on both the monoallelic data set and the multiallelic data set.

According to a further preferred embodiment, the invention provides three or more training cycles. One training cycle of said three or more cycles being a supervised learning period, in which the model is trained on both the monoallelic data set and the multiallelic data set to predict the complete sequence of amino acids being presented by a specific set of alleles. One training cycle of said three or more cycles being a burn-in period, during which only samples derived from monoallelic data sets are used, in order for the model to learn specific peptide-HLA relationships. One cycle of said three or more cycles being a generalization period, during which the multiallelic data set is used to generalize the model, thereby learning to patient data.

According to a preferred embodiment, the epitope sequences of the inputs of each input-output pair of the positive data set are obtained by mass spectrometry. New technological developments have allowed for the increased availability of mass spectrometry-derived lists of peptides that are actually bound to MHC molecules at the cell surface. These lists are called "ligandomes". Within the context of this text, the term "ligandome" is to be understood as the complete set of molecular ligands for proteins in cells and organisms. Preferably, the positive set of input-output pairs is constructed from ligandome data from training cells.

Preferably, the deep learning model according to the present invention is at least one of a deep semantic similarity model, a convolutional deep semantic similarity model, a recurrent deep semantic similarity model, a deep relevance matching model, a deep and wide model, a deep language model, a transformer network, a long short-term memory network, a learned deep learning text embedding, a learned named entity recognition, a Siamese neural network, an interaction Siamese network or a lexical and semantic matching network, or any combination thereof.

Preferably, training the deep learning model comprises determining a score function. More preferably, wherein the score function is one or more of squared error score function, average score function or maximum score function. Preferably, the score function is constructed as being the sum of squared errors between the probabilities being output by the model and HLA-neoepitope relationship information associated to the training data set. Furthermore, this can be implemented by using the score 0 and 1. These scores represent the values ascribed to the ground truth in the training data set, of 'not presented' (=0) and 'presented' (=1).

In a further embodiment of the invention, the coefficients of the model are adjusted at every training step in order to minimize the score function. A neural network is made up of neurons connected to each other; at the same time, each connection of our neural network is associated with a weight that dictates the importance of this relationship in the neuron when multiplied by an input value. In order for neural networks to learn, weights associated with neuron connections must be updated after forward passes of data through the network. These weights are adjusted to help reconcile the differences between the actual and predicted outcomes for subsequent forward passes, often through a process called backpropagation.

Preferably, the deep learning model according to the invention is a sequence-to-sequence model. "Sequence-to-Sequence model (seq2seq)," as used herein, refers to a term known in the state of the art, also referred to as an Encoder Decoder model, that should preferably be understood as a model wherein an encoder reads an input sequence and outputs a single vector and wherein the decoder reads that vector to produce an output sequence. Such model thus aims to map a fixed- and/or unfixed-length input with a fixed- and/or unfixed-length output where the length of the input and output may differ. The use of a seq2seq approach, in which HLA alleles are modeled by the amino acid sequence of specific, functionally relevant sections of their entire structure, has the advantage of being able to extrapolate and predict the presentation likelihood of a neoepitope to HLA alleles that the model has not been trained for. Most preferably, the seq2seq model is a transformer network.

According to a preferred embodiment, the invention provides processing the input of a pair of a plurality of input-output pairs into an embedded input numerical vector by converting the corresponding entry of an epitope sequence using a neoepitope embedder and positional encoder. The embedded input numerical vector comprising information regarding a plurality of amino acids that make up the epitope sequence of the corresponding entry and set of positions of the amino acids in the epitope sequence. According to a further preferred embodiment, the invention provides processing the output of the pair into an embedded output numerical vector by converting the corresponding entry of the peptide sequence of the alpha-chain using an allele embedder and positional encoder. The embedded output numerical vector comprising information regarding the plurality amino acids that make up the peptide sequence of the corresponding entry and a set of positions of the amino acids in the peptide sequence. The embedders and encoders discussed hereabove, allow conversion of input and outputs of the deep learning model are to the appropriate format, prior and after processing, during training, validation or use.

Most preferably, the deep learning model is a transformer network or transformer. Transformer networks were developed to solve the problem of sequence transduction, or neural machine translation. Meaning, any task that transforms or matches an input sequence to an output sequence. For models to perform sequence transduction, it is necessary to have some sort of memory. It needs to figure out dependencies and connections, including long-range connections, between inputs. These transformer neural networks make use of the concept of self-attention and are able to replace earlier approaches of long short-term memory (LSTM) or convolutional neural networks (CNN), that used attention between encoder and decoder of the model. A self-attention mechanism allows the inputs of a model to interact with each other and find out to which element or part they should pay more attention to. The outputs are aggregates of these interactions and attention scores.

More in detail, an attention function can be described as mapping a query, i.e. a sequence, and a set of key-value pairs to an output, where the query (q), keys (k), values (v), and output are all vectors. The keys and values can be seen as the memory of the model, meaning all the queries that have been processed before. A score is calculated to determine self-attention of a token, i.e. an amino acid, in a sequence. Each token of the sequence needs to be scored against the token for which self-attention calculation is desired. That score determines how much focus needs to be placed on other parts of the sequence as a token is encoded at a certain position. That score is calculated by taking the dot product of the query vector with the key vector of the respective token that is scored. By adopting scaled dot product attention, the output is computed as a weighted sum of the values, where the weight assigned to each value is determined by dot product of the query with all the keys.

There are different motivations for the use of self-attention methods. A main advantage of using transformer-style neural networks is that the encoder self-attention can be parallelized, thus decreasing overall model training time. Another one is the path length between long-range dependencies in the network. Learning long-range dependencies is a key challenge in many sequence transduction tasks. One key factor affecting the ability to learn such dependencies is the length of the paths that forward and backward signals have to traverse in the network. The shorter these paths between any combination of positions in the input and output sequences, the easier it is to learn long-range dependencies.

According to a preferred embodiment, the transformer network comprises an encoder and decoder,
the encoder comprising:
   ∘ a neoepitope embedder;
   ∘ a positional encoder;
   ∘ one or more sequence encoders, each comprising two sublayers:
      i. a multi-headed self-attention sublayer;
      ii. a feed-forward sublayer;
the decoder comprising:
   ∘ one or more sequence decoders, each comprising three sublayers:
      i. a multi-headed self-attention sublayer;
      ii. a multi-headed encoder-decoder attention sublayer;
      iii. a feed-forward sublayer;
   ∘ an HLA sequence embedder;
   ∘ a probability generator, comprising:
      i. a linear projector;
      ii. a softmax layer.

"Embedders" turn each input into a vector or tensor using an embedding algorithm. This transformation is necessary because many machine learning algorithms, including deep neural networks, require their input to be vectors of continuous values since they won't work on strings of plain text. Using an embedder gives the advantage of dimensionality reduction and contextual similarity. By reducing the dimensionality of your feature or data set, the model accuracy improves, the algorithm trains faster, less storage space is required and redundant features and noise are removed. The degree of similarity between a pair of inputs can be computed by some similarity or distance measure that is applied to the corresponding pairs of vectors, giving a more expressive representation of the data.

In transformers, self-attention ignores the position of tokens within the sequence. However, the position and order of tokens, i.e. amino acids, are essential parts of a sequence. To overcome this limitation, transformers explicitly add "positional encodings", which are pieces of information that are added to each token about their position in the sequence. Both input and output embedded sequences are position-encoded to allow for the self-attention process to correctly infer position-related interdependencies. These are added to the input or output embedding before the sum goes into the first attention layer.

A "sequence encoder" is composed of a stack of several identical layers. Each layer has two sublayers. The first is a "multi-head self-attention" mechanism, and the second is a simple "feed-forward network". Rather than only computing the attention once, the multi-head mechanism runs through the scaled dot product attention multiple times in parallel. The independent attention outputs are simply concatenated and linearly transformed into expected dimensions. This expands the model's ability to focus on different positions. The outputs of the self-attention layer are fed to a simple feed-forward neural network, in which the information moves further in only one direction. A residual connection or shortcut is employed around each of the two sublayers, which allows the model to use fewer layers in the initial training stages and thereby simplifies the network. Each layer ends with normalization over the sum of its own output and the residual connection. The "sequence decoder" is very similar to the encoder but has an extra "multi-headed encoder-decoder attention sublayer". The encoder-decoder sublayer is different from the encoder or decoder attention sublayers. Unlike multi-head self-attention, the encoder-decoder attention sublayer creates its query matrix from the layer beneath it, which is the decoder self-attention, and takes the keys and values matrix from the output of the encoder layer. This helps the decoder focus on appropriate places in the input sequence.

The decoder output is converted to predicted next-token probabilities by using a "linear projection" or transformation and a "softmax function" or "softmax layer". A linear projection layer reduces the dimensionality of the data, as well as the number of network parameters. Softmax layers are multi-class operations, meaning they are used in determining probability of multiple classes at once. Since the outputs of a softmax function can be interpreted as a probability, i.e. they must sum up to 1, a softmax layer is typically the final layer used in neural network functions.

According to a preferred embodiment, training of the deep learning model comprises a plurality training steps, each training step comprising processing of a pair of the plurality of input-output pairs according to the steps of:
∘ processing the input of the pair into an embedded input numerical vector by converting the corresponding entry of an epitope sequence using a neoepitope embedder and positional encoder, the embedded input numerical vector comprising information regarding a plurality of amino acids that make up the epitope sequence of the corresponding entry and set of positions of the amino acids in the epitope sequence;
∘ processing the output of the pair into an embedded output numerical vector by converting the corresponding entry of the peptide sequence of the alpha-chain using an allele embedder and positional encoder, the embedded output numerical vector comprising information regarding the plurality amino acids that make up the peptide sequence of the corresponding entry and a set of positions of the amino acids in the peptide sequence;
∘ processing the embedded input numerical vector into an encoded input numerical vector using at least one sequence encoder comprising a multi-headed self-attention sublayer and a feed-forward sublayer, the encoded input numerical vector comprising information regarding a feature of the epitope sequence of the corresponding entry of the epitope sequence;
∘ processing the embedded output numerical vector into an output attention numerical vector using a multi-headed self-attention sublayer, the output attention numerical vector comprising information regarding interdependencies of the plurality of amino acids that make up the peptide sequence of the corresponding entry of the peptide sequence of the alpha-chain;
∘ processing the encoded input numerical vector and corresponding output attention vector into a correlation numerical vector using a multi-headed encoder-decoder attention sublayer and feed-forward sublayer, the correlation numerical vector comprising correlation information between the encoded input numerical vector and the corresponding output attention vector; and
∘ processing the correlation numerical vector into a probability of correspondence between the embedded input numerical vector and the embedded output numerical vector using a probability generator.

In a further embodiment, the embedding of both the input of the pair, the epitope sequence, and of the output of the pair, the HLA peptide sequence, may follow one of different modalities.

According a first possible modality, each amino-acid position is one-hot encoded, meaning that it is transformed into a 1 × 20 vector, as there are 20 canonical amino acids. At each position of the vector is a 0 (zero), except in one position where a 1 (one) is present. This latter position represents the actual amino-acid present. In this manner, for instance, a 9mer is transformed into a 9 x 20 matrix where only 9 positions are 1, while all other positions are 0.

According to a second possible modality, each amino-acid is individually tokenized, meaning that an amino-acid - to numeric value dictionary is constructed, wherein every amino-acid is represented by a numeric value. For instance, proline is represented as 1, while valine is represented as 2, .... In this manner, a 9mer is transformed into a vector with length of 9 numbers.

According to a third possible modality, each amino-acid is replaced by an embedding vector of n numerical values. These n numerical values relate to specific characteristics of the amino-acid, which may be physical, chemical or otherwise defined. As a preferred example, an amino-acid is embedded by the values of its *n* principal components derived from a set of physico-chemical properties/characteristics. Therefore, a 9mer is in this example transformed into a 9 x n numerical matrix.

The three possible embedding modalities can be performed directly on individual amino-acid position, wherein 1 amino-acid is embedded to 1 embedding vector. In another or further modality, for the embedding of both the epitope sequences (inputs) and HLA sequences (outputs), the sequences can be divided into strings having a length of more than 1. In this manner, instead of considering individual amino-acids, k-mers are considered.

According to a further preferred embodiment, the processing of a pair of the plurality of input-output pairs further comprises the step of:
o obtaining a data point of a score function for training by comparing the probability of correspondence between the embedded input numerical vector and the embedded output numerical vector to corresponding relation information associated to the training data set;
o adjusting a parameter associated to the deep learning model in order to optimize said score function;
preferably wherein the score function is one or more of squared error score function, average score function or maximum score function.

In an embodiment, the score function may be a binary cross-entropy loss function.

In an embodiment of the invention, as explained before, positive input-output pairs can be assigned different weights, preferably depending on the frequency of occurrence in the mass spectrometry data used to build the positive training set. The weights modify the impact the pairs have on the training of the deep learning model. A larger weight will lead to a larger adjustment of parameters associated to the deep learning model when training the model with said input-output pair.

According to another preferred embodiment, the transformer network comprises an encoder but no decoder. In this network, both input epitope sequence and input HLA sequence embedded vectors are processed as a single vector. To indicate whether a value of the input embedding vector relates to either a neoepitope or an HLA, a type of masking is performed. This means that for instance the sign of the numerical values associated with the epitope input is changed while said sign associated with the HLA input is not changed. In addition, in this network model, custom separator values are inserted at various positions of the input embedded vectors, in particular at the start and/or at the end of the vectors, as well as in between the epitope-related values and the HLA-related values. In this way, it is possible to have both input sequences processed as a single vector, while still being able to differentiate between both input sequences.

According to a further preferred embodiment, after training of the model, one or more of the following are obtained:
- a set of coefficients that can be used to reproduce its function given the correct structure;
- a set of parameters describing all aspects of the training of the model;
- a structure scheme that can be used to regenerate the model for inference/testing;
- a dictionary of the HLA's seen during model training.

According to an embodiment, the invention provides a method wherein other semi-independent models can be trained in relation to the central used architecture to take into account other relevant biological parameters. These biological parameters comprise: RNA expression of the gene from which the neoepitope is derived, RNA expression of all the other genes in the sample, expression of noncoding RNAs, Post-Translational Modification state, RNA editing events, immune fractions of every immune cell type, clonality of the sample, confidence score of all genome-altering events, peptide-MHC binding affinity as predicted by other tools, peptide-MHC complex stability, peptide stability and turnover, neighboring amino-acids within the neoepitope original protein, proteasome activity, and peptide processing activity. The model structure is setup in such a way that any missing data on this list will not prevent the model from outputting a presentation probability.

According to a preferred embodiment, the invention further comprises the steps of:
- training a semi-independent neural network on a semi-independent training data set comprising at least the positive data set of the deep learning model or a variant thereof and an associated prediction-improving parameter training data set, wherein said associated prediction-improving parameter training data set relates to one or more biological parameters of RNA expression of a gene from which the neoepitope is derived, RNA expression of a plurality of genes in a cancerous tissue sample, expression of noncoding RNA sequences, Post-Translational Modification information, RNA editing event information, immune fractions of a plurality of immune cell types, clonality of a cancerous tissue sample, confidence score of a plurality of genome-altering events, peptide-MHC binding affinity, peptide-MHC complex stability, peptide stability and/or turnover, neighbouring amino-acids within the neoepitope sequence, proteasome activity, and peptide processing activity,
   preferably wherein wherein said associated prediction-improving parameter training data set at least relates to neighbouring amino-acids within the neoepitope sequence;
- determining a semi-independent presentation likelihood for each of the set of neoantigens for the peptide sequence of the HLA by means of the trained semi-independent neural network; and
- combining for each of the set of neoantigens the determined semi-independent presentation likelihood and the presentation likelihood obtained by means of the trained model to obtain an overall presentation likelihood;

preferably wherein combining is performed by means of a trained single layer neural network;
preferably wherein the semi-independent neural network is a single layer neural network;
preferably wherein the at least one of exome or whole genome nucleotide sequencing data and transcriptome nucleotide sequencing data from tumour cells associated to said tumour and normal cells of the subject are respectively obtained from a cancerous tissue sample and a healthy tissue sample of the subject.

According to an embodiment, training of all the sublayers are performed by using an Adam-type optimization algorithm. Optimizers are algorithms or methods used to change the attributes of your neural network such as weights and learning rates in order to reduce the losses or errors and help to get results faster. The algorithm leverages the power of adaptive learning rates methods to find individual learning rates for each parameter. Adam uses estimations of first and second moments of gradient to adapt the learning rate for each weight of the neural network.

According to an embodiment, the deep learning model, preferably the transformer network, is trained for 5 epochs of 5-fold cross-validation. k-fold cross-validation is easy to understand, easy to implement, and results in skill estimates, for a model on new data, that generally have a lower bias than other methods. There is a bias-variance trade-off associated with the choice of k in k-fold cross-validation.

Performing k-fold cross-validation using k = 5, yields test error rate estimates that suffer neither from excessively high bias nor from very high variance.

"Epoch," as used herein, refers to a term known in the state of the art, that should preferably be understood as an indication of the number of passes through an entire training dataset a machine learning algorithm completes. One epoch is one cycle through the full training dataset.

"K-fold cross-validation," as used herein, refers to a term known in the state of the art, that should preferably be understood as a statistical method to estimate the skill of machine learning models. This approach involves repeatedly randomly dividing a set of observations into k groups, or folds, of approximately equal size. The first fold is treated as a validation set, and the method is fit on the remaining k-1 folds. The results of a k-fold cross-validation run is often summarized with the mean of the model skill scores. It is also good practice to include a measure of the variance of the skill scores, such as the standard deviation or standard error.

The invention is further described by the following non-limiting examples which further illustrate the invention, and are not intended to, nor should they be interpreted to, limit the scope of the invention.

### EXAMPLES

### Example 1:

The present example pertains to training of a sequence-to-sequence transformer model according to the present invention.

The sequence-to-sequence transformer model has the following architecture:
- encoder:
   ∘ a neoepitope embedder;
   ∘ a positional encoder;
   ∘ one or more sequence encoders, each comprising two sublayers:
      i. a multi-headed self-attention sublayer;
      ii. a feed-forward sublayer;
- decoder:
   ∘ one or more sequence decoders, each comprising three sublayers:
      i. a multi-headed self-attention sublayer;
      ii. a multi-headed encoder-decoder attention sublayer;
      iii. a feed-forward sublayer;
   ∘ an HLA sequence embedder;
   ∘ a probability generator, comprising:
      i. a linear projector;
      ii. a softmax layer.

The hereabove described sequence-to-sequence transformer model is trained by processing sets of positive and of negative input-output pairs through the model.

A positive set of input-output pairs is constructed from ligandome data from monoallelic human cell lines or multiallelic human tissue (healthy or cancerous). Each positive input consists in the sequence of an epitope (8 to 15 amino acids) that was shown to be present at the cell surface in a given dataset. Each associated positive output is made of the concatenated amino-acid sequence of the alpha chains of the HLA allele(s) expressed by the cell in the same dataset (71 amino-acids).

A negative set of input-output pairs is constructed from the human proteome. Each input is a random 8- to 15-mer sequence from the human proteome not present in any ligandome dataset. Each associated output is a concatenation of the sequence of the alpha chains of a random set of HLA allele(s) present in the positive dataset.

Each training input-output pair is processed through the model as follows:
- The input peptide is padded up to a length of 15 with "." tokens if necessary, and the resulting sequence is then embedded by the neoepitope embedder into a 21*15 one-hot tensor
- The sequence-based model embeds every HLA by the allele embedder into a 21 * 71 one-hot tensor according to the sequence of its two peptide-interacting alpha-helices.
- Both input and output embedded sequence are then position-encoded to allow for the self-attention process to correctly infer position-related interdependencies.
- The embedded input sequence is sequentially processed by every sequence encoder. The self-attention sublayers learn within-peptide interdependencies and the feed-forward sublayers process the input embedding accordingly.
- The result of this encoding process is a fixed-dimension, feature-representative encoding of the input neopeptide.
- The embedded HLA sequence input is in turn processed and combined with the encoded neoepitope input sequentially in every decoder, progressively forming the embedded output sequence. The self-attention sublayers learn within-allele interdependencies; the peptide-attention sublayers correlate the encoded peptide representation with the embedded output, and the feed-forward sublayers apply modifications to the embedded output accordingly. In this step the correspondence between input and output is established. It should be noted that the attention sublayers, which allow for detection of within-sequence interdependency, significantly improve the overall prediction power of the model.
- Finally, the embedded output is processed through the generator to output a probability of correspondance between embedded input and embedded output, representing a probability of presentation (0 to 1, 1 being the highest probability).
- A score function is constructed as being the sum of squared errors between the probabilites output by the model and the actual HLA-peptide relationship (0: peptide was not presented at the surface of cells expressing this allele, ie peptide was part of the aforementioned negative dataser - 1: peptide was presented at the surface of cells expressing this allele, ie was part of the aforementioned positive dataset). Other ways of aggregating the data are possible, such as considering the average score function or the maximum score function.
- At every training step, ie with every new input-output pair processing, the coefficients of the model are adjusted to minimize the score function thus defined.

The model is trained as follows:
- The model is trained for 5 epochs of 5-fold cross-validation.
- Training of this model follows the following steps: First, the model is trained on ALL samples to simply predict, amino-acid by amino-acid, the complete sequence of amino-acid being presented by a specific set of alleles (self-supervised learning). Then, only samples derived from monoallelic HLA datasets (e.g. from monoallelic cell lines) are used for training ("burn-in" period), in order for the model to learn specific peptide-HLA relationships. Finally, HLA multiallelic instances are used for training in order to generalize model learning to actual patient data.
- The training of all layers of the model are done using an ADAM-type optimizer.

At the end of training, the model outputs a set of coefficients that can be used to reproduce its function given the correct structure, a set of parameters describing all aspects of the training of the model, a structure scheme that can be used to regenerate the model for inference/testing, and a dictionary of the HLAs seen during model training.

### Example 2:

The present example pertains to use of a trained model according to example 1 in a workflow according to the present invention.

The embodiment provides a workflow for predicting likelihood of presentation at a cancer cell surface of a variable-length neoepitope given a set of HLA alleles expressed by the cell.

The workflow uses a sequence-to-sequence transformer model. Such model allows extrapolation and prediction of presentation likelihoods of the neoepitope to any HLA allele, even if it has not been trained on it.

The workflow is as follows:
- First, neopeptides are discovered using next-generation sequencing data on a cancer biopsy. Both DNA and RNA sequencing data are used to extract a set of aberrant genomic events potentially delivering neoepitopes.
- These events are given a confidence score based on the number of sequencing reads supporting them and their pervasiveness in the genome, and epitopes from the highest-confidence events are chosen for follow-up steps.
- The HLA make-up of the biopsy is also assessed using the same genomic data.
- The sequence of the chosen peptides is provided to the trained model along with the sequence of known HLAs.
- The model calculates a probability of peptide presentation for each HLA of the provided set and output an overall peptide probability based on these individual values

Additionally, the workflow may or may not comprise the step of refining the probability prediction by providing other biological parameters to the model, such as such as RNA expression levels, MHC binding likelihood or neoepitope protein context.

### Example 3:

The present example pertains to alternative implementations of the transformer model according example 1.

As described hereabove in example 1, the input neoepitope sequence is padded up to a length of 15 with "." tokens if necessary and the resulting sequence is then embedded by the neoepitope embedder into a 21 × 15 one-hot tensor. The model of example 1 thus requires the sequence to be within a correct length range. However, the model can also be implemented in order to allow for any length epitopes and HLAs. Similarly, the model may be implemented in order to allow for a variable-length embedding. Furthermore, the model may be implemented in order to allow for embedding onto a different size matrix, up to 300 x 15.

As described hereabove in example 1, the model is sequence-based and embeds every HLA by the allele embedder into a 21 * 71 one-hot tensor according to the sequence of its two peptide-interacting alpha-helices. Alternatively, the model can process associated HLAs as a categorical encoding. Categorical encoding refers to transforming a categorical feature into one or multiple numeric features. Every HLA is thereby encoded according to a central repository regrouping all HLA sequences known at the time the model was built. Alternatively, the model can also be non-sequence-based. HLAs are thereby one-hot encoded based on their previous central repository encoding. Associated HLA sequences are processed one by one. As such, a specific neoepitope will be processed once for each HLA sequence it was found to be associated with in a specific sample. This model will not be able to output a prediction if the HLA allele amino acid sequence is unknown. This is a remote, but real possibility for some rare HLA alleles.

### Example 4:

The present example pertains to use of the workflow according to example 2 for determining a treatment for a subject.

The determining of a treatment is as follows:
- selecting a sub-set of the identified set of neoantigens based on the determined presentation likelihoods to obtain a sub-set of selected neoantigens,
   wherein the sub-set is obtained by comparing the presentation likelihood of each of the set of neoantigens to a threshold value and wherein a neoantigen is added to said sub-set if the associated presentation likelihood exceeds said threshold value; and
- identifying one or more T-cells that are antigen-specific for at least one of the neoantigens in said sub-set.

### Example 5:

The present example pertains to an improved model comprising the sequence-to-sequence transformer model according to example 1 and one or more semi-independent models to said transformer model. The improved model can used in the workflow according to example 2 for determining a treatment for a subject.

According to the present example, a plurality of semi-independent single layer neural network models are trained in relation to the central transformer architecture to take into account other relevant biological parameters. Accordingly, each of said plurality of semi-independent models is trained by training a single layer neural network on a semi-independent training data set comprising the training data set of the sequence-to-sequence transformer model and an associated prediction-improving parameter training data set. By taking into account parameters from the prediction-improving parameter training data set, overall prediction accuracy is improved.

The parameter training data set of each of the plurality of semi-independent single layer neural network model relates to one or more biological parameters of RNA expression of a gene from which the neoepitope is derived, RNA expression of all genes in the cancerous tissue sample except for the gene from which the neoepitope is derived, expression of noncoding RNA sequences, Post-Translational Modification state, RNA editing events, immune fractions of every immune cell type, clonality of the cancerous tissue sample, confidence score of all genome-altering events, peptide-MHC binding affinity as predicted by other tools, peptide-MHC complex stability, peptide stability and turnover, neighbouring amino-acids within the neoepitope original protein, proteasome activity, and peptide processing activity.

After training of each of semi-independent models, a semi-independent presentation likelihood is determined for each of the set of neoantigens for the peptide sequence of the HLA by means of the trained semi-independent neural network. This determined semi-independent presentation likelihood is then combined for each of the set of neoantigens with the determined semi-independent presentation likelihood and the presentation likelihood obtained by means of the trained model to obtain an overall presentation likelihood. According to the present example, combining is performed by means of a trained single layer neural network.

### Example 6:

The example pertains to a comparison between a model according to the present invention and prior art algorithms, the EDGE algorithm and the MHCflurry algorithm.

A sequence-to-sequence transformer model according to the present invention was developed and trained on:
- a positive data set comprising 326.297 publicly available input-output pairs wherein each pair comprises an entry of an epitope sequence as input, said epitope sequence being identified or inferred from a surface bound or secreted HLA/peptide complex encoded by a corresponding HLA allele expressed by a training cell, wherein each pair further comprises an entry of a peptide sequence of an alpha-chain encoded by the corresponding HLA allele as output; publicly available from Abelin et al., 2017; Bulik-Sullivan et al., 2019; di Marco et al., 2017; Sarkizova et al., 2019; and Trolle et al., 2016; and
- a negative data set comprising 652.594 input-output pairs, each pair comprising an entry of a peptide sequence as input, wherein said peptide sequence is a random sequence of a human proteome and wherein each pair further comprises a peptide sequence encoded from a random HLA allele as output.

The model was then tested on test dataset comprising:
- 729 positive pairs, which were chosen from the published test dataset of the EDGE algorithm (Bulik-Sullivan et al., 2019), and
- 1.822.500 negative pairs, each pair comprising an entry of a peptide sequence as input, wherein said peptide sequence is a random sequence of a human proteome and wherein each pair further comprises a peptide sequence encoded from a random HLA allele as output.

Care was taken to not include pairs in the test dataset which were already included in the training phase of the model.

Test dataset precision-recall curve were generated. Precision is measured as the proportion of called positive epitopes that were truly presented, while recall measures the proportion of truly positive epitopes that were accurately called positive. As such, the precision recall curve is a good measure of the ability of a model to accurately call desirable positive outcomes without making mistakes. The better the model, the more the precision-recall curve skews towards the top right corner.

Results are shown in Figure 1A, wherein the results of the transformer model according to the present invention are shown in blue (skewing most towards the top right corner), while the results of the EDGE algorithm are shown in black. In addition, the (substantially flat) green line represents the best precision achieved by the affinity-based model MHCflurry.

From the results, it is clear that the model according to the present invention outperforms close prior art algorithm EDGE, as well as current state-of-the-art industry methods like MHCflurry on the same test dataset.

### Example 7:

This example pertains to the ability of a model according to the present invention for extrapolation and prediction.

As a sequence-to-sequence algorithm, the model derives its predictive power not from categorical data, but from comparing and drawing correlations between two sequences. This implies that it is able to make predictions for HLA alleles for which no training data was available, provided their protein sequence is known.

This ability for extrapolation/prediction is a true advantage, considering that obtaining novel training data is a long and costly process.

To test this capability, the model was trained as in example 6, and a new test dataset was constructed from 2.039 positive pairs uniquely associated with the HLA-A*74:02 allele, for which no data was present in the training set, along with 5.097.500 negative pairs each pair comprising an entry of a peptide sequence as input, wherein said peptide sequence is a random sequence of a human proteome and wherein each pair further comprises a peptide sequence encoded from a random HLA allele as output.

Results are shown in Figure 1B. The precision-recall curve clearly indicates that the model according to the present invention has a very good predictive power even on this previously unseen allele.

## Claims

1. Computer-implemented method for determining presentation likelihoods of a set of neoantigens by a tumour cell of a tumour of a subject, the method comprising the steps of:
- obtaining at least one of exome or whole genome nucleotide sequencing data and transcriptome nucleotide sequencing data from tumour cells associated to said tumour and normal cells of the subject;
- obtaining a set of aberrant genomic events associated to said tumour by comparing the exome and/or whole genome nucleotide sequencing data and the transcriptome nucleotide sequencing data from the tumour cells to the exome and/or whole genome nucleotide sequencing data and the transcriptome nucleotide sequencing data from the normal cells;
- obtaining data representing peptide sequences of each of a set of neoantigens identified based at least in part on said set of aberrant events, wherein the peptide sequence of each neoantigen comprises at least one alteration which makes it distinct from a corresponding wild-type peptide sequence identified from the normal cells of the subject;
- obtaining data representing a peptide sequence of a human leukocyte antigen (HLA) based on the tumour exome and/or whole genome nucleotide sequencing data and the transcriptome nucleotide sequencing data from the tumour cells;
- training a deep learning model on a training data set comprising a positive data set, wherein the positive data set comprises a plurality of input-output pairs, wherein each pair comprises an entry of an epitope sequence as input, said epitope sequence being identified or inferred from a surface bound or secreted HLA/peptide complex encoded by a corresponding HLA allele expressed by a training cell, wherein each pair further comprises an entry of a peptide sequence of an alpha-chain encoded by the corresponding HLA allele as output; and
- determining a presentation likelihood for each of the set of neoantigens for the peptide sequence of the HLA by means of the trained model.

2. Method according to preceding claim 1, further comprising the steps of:
- associating a confidence score to each of said set of aberrant genomic events based at least in part on a number of sequencing reads of the sequencing data supporting each associated aberrant genomic event;
- obtaining a sub-set of aberrant genomic events by comparing the confidence score of each aberrant genomic event of said set of aberrant genomic events to a threshold value, wherein an event is added to said sub-set if the associated confidence score exceeds said threshold value;
wherein said set of neoantigens are identified based at least in part on said sub-set of aberrant genomic events.

3. Method according to any of preceding claims 1 or 2, wherein the positive data set comprises a monoallelic and multiallelic data set, wherein the monoallelic data set comprises input-output pairs obtained from a training cell from a monoallelic cell line and wherein the multiallelic data set comprises input-output pairs obtained from a training cell from a multiallelic tissue.

4. Method according to preceding claim 3, wherein the training of the deep learning model comprises two or more training cycles, wherein each training cycle comprises a plurality of training steps, wherein each training step comprises processing a pair of the plurality of input-output pairs, wherein one of said two or more training cycles comprises training the deep learning model on the monoallelic data set and wherein one of said two or more training cycles comprises training the deep learning model on both the monoallelic data set and the multiallelic data set.

5. Method according to any of preceding claims 1 to 4, wherein the training data set for training the deep learning model further comprises a negative data set comprising a plurality of input-output pairs, each pair comprising an entry of a peptide sequence as input, wherein said peptide sequence is a random sequence of a human proteome and wherein each pair further comprises a peptide sequence encoded from a random HLA allele as output.

6. Method according to any of preceding claims 1 to 5, wherein the deep learning model is at least one of a deep semantic similarity model, a convolutional deep semantic similarity model, a recurrent deep semantic similarity model, a deep relevance matching model, a deep and wide model, a deep language model, a transformer network, a long short-term memory network, a learned deep learning text embedding, a learned named entity recognition, a Siamese neural network, an interaction Siamese network or a lexical and semantic matching network, or combinations thereof.

7. Method according to any of preceding claims 1 to 6, wherein the deep learning model is a transformer network.

8. Method according to any of preceding claims 1 to 7, wherein the training of the deep learning model comprises a plurality training steps, each training step comprising processing of a pair of the plurality of input-output pairs according to the steps of:
∘ processing the input of the pair into an embedded input numerical vector by converting the corresponding entry of an epitope sequence using a neoepitope embedder and positional encoder, the embedded input numerical vector comprising information regarding a plurality of amino acids that make up the epitope sequence of the corresponding entry and a set of positions of amino acids in the epitope sequence;
∘ processing the output of the pair into an embedded output numerical vector by converting the corresponding entry of the peptide sequence of the alpha-chain using an allele embedder and positional encoder, the embedded output numerical vector comprising information regarding the plurality amino acids that make up the peptide sequence of the corresponding entry and a set of positions of the amino acids in the peptide sequence;
∘ processing the embedded input numerical vector into an encoded input numerical vector using at least one sequence encoder comprising a multi-headed self-attention sublayer and a feed-forward sublayer, the encoded input numerical vector comprising information regarding a feature of the epitope sequence of the corresponding entry of the epitope sequence;
∘ processing the embedded output numerical vector into an output attention numerical vector using a multi-headed self-attention sublayer, the output attention numerical vector comprising information regarding interdependencies of the plurality of amino acids that make up the peptide sequence of the corresponding entry of the peptide sequence of the alpha-chain;
∘ processing the encoded input numerical vector and corresponding output attention vector into a correlation numerical vector using a multi-headed encoder-decoder attention sublayer and feed-forward sublayer, the correlation numerical vector comprising correlation information between the encoded input numerical vector and the corresponding output attention vector; and
∘ processing the correlation numerical vector into a probability of correspondence between the embedded input numerical vector and the embedded output numerical vector using a probability generator.

9. Method according preceding claims 8, wherein the processing of a pair of the plurality of input-output pairs further comprises the step of:
∘ obtaining a data point of a score function for training by comparing the probability of correspondence between the embedded input numerical vector and the embedded output numerical vector to corresponding relation information associated to the training data set;
∘ adjusting a parameter associated to the deep learning model to optimize said score function;
preferably wherein the score function is one or more of a squared error sum score function, average score function or maximum score function.

10. Method according to any of preceding claims 7 to 9, wherein the transformer network comprises an encoder and a decoder;
the encoder comprising:
∘ a neoepitope embedder;
∘ a positional encoder;
∘ one or more sequence encoders, each comprising two sublayers:
i. a multi-headed self-attention sublayer;
ii. a feed-forward sublayer;
the decoder comprising:
∘ one or more sequence decoders, each comprising three sublayers:
i. a multi-headed self-attention sublayer;
ii. a multi-headed encoder-decoder attention sublayer;
iii. a feed-forward sublayer;
∘ an HLA sequence embedder;
∘ a probability generator, comprising:
i. a linear projector;
ii. a softmax layer.

11. Method according to any of preceding claims 1 to 10, further comprising the steps of:
- training a semi-independent neural network on a semi-independent training data set comprising at least the positive data set of the deep learning model or a variant thereof and an associated prediction-improving parameter training data set, wherein said associated prediction-improving parameter training data set relates to one or more biological parameters of RNA expression of a gene from which the neoepitope is derived, RNA expression of a plurality of genes in a cancerous tissue sample, expression of noncoding RNA sequences, Post-Translational Modification information, RNA editing event information, immune fractions of a plurality of immune cell types, clonality of a cancerous tissue sample, confidence score of a plurality of genome-altering events, peptide-MHC binding affinity, peptide-MHC complex stability, peptide stability and/or turnover, neighbouring amino-acids within the neoepitope sequence, proteasome activity, and peptide processing activity, preferably wherein wherein said associated prediction-improving parameter training data set at least relates to neighbouring amino-acids within the neoepitope sequence;
- determining a semi-independent presentation likelihood for each of the set of neoantigens for the peptide sequence of the HLA by means of the trained semi-independent neural network; and
- combining for each of the set of neoantigens the determined semi-independent presentation likelihood and the presentation likelihood obtained by means of the trained model to obtain an overall presentation likelihood;
preferably wherein combining is performed by means of a trained single layer neural network;
preferably wherein the semi-independent neural network is a single layer neural network.

12. Computer system for determining presentation likelihoods of a set of neoantigens by a tumour cell of a tumour of a subject, the computer system configured for performing the computer-implemented method according to any one of the preceding claims 1 to 11.

13. Computer program product for determining presentation likelihoods of a set of neoantigens by a tumour cell of a tumour of a subject, the computer program product comprising instructions which, when the computer program product is executed by a computer, cause the computer to carry out the computer-implemented method according to any one of preceding claims 1 to 11.

14. Use of the computer-implemented method according to any of preceding claims 1 to 11 and/or the computer system according to preceding claim 12 and/or the computer program product according to preceding claim 13, for determining a treatment for the subject.

15. Use according to preceding claim 14, wherein the determining of a treatment comprises: selecting a sub-set of the identified set of neoantigens based on the determined presentation likelihoods to obtain a sub-set of selected neoantigens, preferably wherein the sub-set is obtained by comparing the presentation likelihood of each of the set of neoantigens to a threshold value, wherein a neoantigen is added to said sub-set if the associated presentation likelihood exceeds said threshold value; and identifying one or more T-cells that are antigen-specific for at least one of the neoantigens in said sub-set.

## Patentansprüche

1. Computerimplementiertes Verfahren zum Bestimmen von Präsentationswahrscheinlichkeiten eines Satzes von Neoantigenen durch eine Tumorzelle eines Tumors eines Subjekts, wobei das Verfahren die folgenden Schritte umfasst:
- Gewinnen von mindestens einem von Exom- oder Vollgenom-Nukleotidsequenzierungsdaten und Transkriptom-Nukleotidsequenzierungsdaten von mit dem Tumor assoziierten Tumorzellen und von normalen Zellen des Subjekts,
- Gewinnen eines Satzes von mit dem Tumor assoziierten aberranten genomischen Ereignissen durch Vergleichen der Exom- und/oder Vollgenom-Nukleotidsequenzierungsdaten und der Transkriptom-Nukleotidsequenzierungsdaten aus den Tumorzellen mit den Exom- und/oder Vollgenom-Nukleotidsequenzierungsdaten und den Transkriptom-Nukleotidsequenzierungsdaten aus den normalen Zellen,
- Gewinnen von Daten, die Peptidsequenzen von jedem eines Satzes von Neoantigenen repräsentieren, die zumindest teilweise basierend auf dem Satz aberranter Ereignisse identifiziert wurden, wobei die Peptidsequenz jedes Neoantigens mindestens eine Veränderung umfasst, die sie von einer entsprechenden Wildtyp-Peptidsequenz unterscheidet, die aus den normalen Zellen des Subjekts identifiziert wurde,
- Gewinnen von Daten, die eine Peptidsequenz eines humanen Leukozyten-Antigens (HLA) repräsentieren, basierend auf den Exom- und/oder Vollgenom-Nukleotidsequenzierungsdaten des Tumors und den Transkriptom-Nukleotidsequenzierungsdaten aus den Tumorzellen,
- Trainieren eines Deep-Learning-Modells an einem Trainingsdatensatz, der einen positiven Datensatz umfasst, wobei der positive Datensatz mehrere Eingabe-Ausgabe-Paare umfasst, wobei jedes Paar einen Eintrag einer Epitopsequenz als Eingabe umfasst, wobei die Epitopsequenz aus einem oberflächengebundenen oder sekretierten HLA/Peptid-Komplex identifiziert oder hergeleitet wird, für den ein entsprechendes HLA-Allel codiert, das von einer Trainingszelle exprimiert wird, wobei jedes Paar ferner einen Eintrag einer Peptidsequenz einer Alpha-Kette, für die das entsprechende HLA-Allel codiert, als Ausgabe umfasst, und
- Bestimmen einer Präsentationswahrscheinlichkeit für jedes des Satzes von Neoantigenen für die Peptidsequenz des HLA mittels des trainierten Modells.

2. Verfahren nach dem vorhergehenden Anspruch 1, ferner die folgenden Schritte umfassend:
- Assoziieren eines Konfidenzwertes (Confidence Score) mit jedem des Satzes aberranter genomischer Ereignisse zumindest teilweise basierend auf einer Anzahl von Sequenzierungs-Reads der Sequenzierungsdaten, die jedes assoziierte aberrante genomische Ereignis belegen,
- Gewinnen eines Teilsatzes aberranter genomischer Ereignisse durch Vergleichen des Konfidenzwertes jedes aberranten genomischen Ereignisses des Satzes aberranter genomischer Ereignisse mit einem Schwellenwert, wobei ein Ereignis dem Teilsatz hinzugefügt wird, wenn der assoziierte Konfidenzwert den Schwellenwert überschreitet,
wobei der Satz von Neoantigenen zumindest teilweise basierend auf dem Teilsatz aberranter genomischer Ereignisse identifiziert wird.

3. Verfahren nach einem der vorhergehenden Ansprüche 1 oder 2, wobei der positive Datensatz einen monoallelischen Datensatz und multiallelischen Datensatz umfasst, wobei der monoallelische Datensatz Eingabe-Ausgabe-Paare umfasst, die von einer Trainingszelle aus einer monoallelischen Zelllinie gewonnen werden, und wobei der multiallelische Datensatz Eingabe-Ausgabe-Paare umfasst, die von einer Trainingszelle aus einem multiallelischen Gewebe gewonnen werden.

4. Verfahren nach dem vorhergehenden Anspruch 3, wobei das Trainieren des Deep-Learning-Modells zwei oder mehr Trainingszyklen umfasst, wobei jeder Trainingszyklus mehrere Trainingsschritte umfasst, wobei jeder Trainingsschritt das Verarbeiten eines Paares der mehreren Eingabe-Ausgabe-Paare umfasst, wobei einer der zwei oder mehr Trainingszyklen das Trainieren des Deep-Learning-Modells an dem monoallelischen Datensatz umfasst und wobei einer der zwei oder mehr Trainingszyklen das Trainieren des Deep-Learning-Modells an sowohl dem monoallelischen Datensatz als auch an dem multiallelischen Datensatz umfasst.

5. Verfahren nach einem der vorhergehenden Ansprüche 1 bis 4, wobei der Trainingsdatensatz zum Trainieren des Deep-Learning-Modells ferner einen negativen Datensatz umfasst, der mehrere Eingabe-Ausgabe-Paare umfasst, wobei jedes Paar einen Eintrag einer Peptidsequenz als Eingabe umfasst, wobei die Peptidsequenz eine Zufallssequenz eines humanen Proteoms ist, und wobei jedes Paar ferner eine Peptidsequenz, die aus einem zufälligen HLA-Allel codiert ist, als Ausgabe umfasst.

6. Verfahren nach einem der vorhergehenden Ansprüche 1 bis 5, wobei das Deep-Learning-Modell mindestens eines von einem tiefen Lernmodell semantischer Nähe (Deep Semantic Similarity Model), einem konvolutionellen tiefen Lernmodell semantischer Nähe (Convolutional Deep Semantic Similarity Model), einem rekurrenten tiefen Lernmodell semantischer Nähe (Recurrent Deep Semantic Similarity Model), einem tiefen Lernmodell eines Relevanzabgleichs (Deep Relevance Matching Model), einem tiefen und weiten Lernmodell (Deep and Wide Model), einem tiefen Sprachenmodell, einem Transformer-Netz, einem Netz mit langem Kurzzeitgedächtnis (Long Short-Term Memory), eine eingelernte Deep-Learning-Texteinbettung, eine eingelernte Eigennamenerkennung (Named Entity Recognition), ein siamesisches neuronales Netz, ein siamesisches Interaktionsnetz oder ein Netz zum lexikalischen und semantischen Abgleich, oder Kombinationen daraus, ist.

7. Verfahren nach einem der vorhergehenden Ansprüche 1 bis 6, wobei das Deep-Learning-Modell ein Transformer-Netz ist.

8. Verfahren nach einem der vorhergehenden Ansprüche 1 bis 7, wobei das Training des Deep-Learning-Modells mehrere Trainingsschritte umfasst, wobei jeder Trainingsschritt das Verarbeiten eines Paares der mehreren Eingabe-Ausgabe-Paare gemäß den folgenden Schritten umfasst:
∘ Verarbeiten der Eingabe des Paares zu einem eingebetteten numerischen Eingabevektor durch Umwandeln des entsprechenden Eintrags einer Epitopsequenz unter Verwendung eines Neoepitop-Einbetters und eines Positions-Encoders, wobei der eingebettete numerische Eingabevektor Informationen bezüglich mehrerer Aminosäuren, aus denen die Epitopsequenz des entsprechenden Eintrags besteht, und eines Satzes von Positionen von Aminosäuren in der Epitopsequenz umfasst,
∘ Verarbeiten der Ausgabe des Paares zu einem eingebetteten numerischen Ausgabevektor durch Umwandeln des entsprechenden Eintrags der Peptidsequenz der Alpha-Kette unter Verwendung eines Allel-Einbetters und eines Positions-Encoders, wobei der eingebettete numerische Ausgabevektor Informationen bezüglich der mehreren Aminosäuren, aus denen die Peptidsequenz des entsprechenden Eintrags besteht, und eines Satzes von Positionen der Aminosäuren in der Peptidsequenz umfasst,
∘ Verarbeiten des eingebetteten numerischen Eingabevektors zu einem codierten numerischen Eingabevektor unter Verwendung mindestens eines Sequenz-Encoders, der eine Teilschicht mit mehrfacher Selbstaufmerksamkeit (Multi-Head Self-Attention Sublayer) und eine Feed-Forward-Teilschicht umfasst, wobei der codierte numerische Eingabevektor Informationen bezüglich eines Merkmals der Epitopsequenz des entsprechenden Eintrags der Epitopsequenz umfasst,
∘ Verarbeiten des eingebetteten numerischen Ausgabevektors zu einem numerischen Ausgabe-Aufmerksamkeits-Vektor unter Verwendung einer Teilschicht mit mehrfacher Selbstaufmerksamkeit (Multi-Head Self-Attention Sublayer), wobei der numerische Ausgabe-Aufmerksamkeits-Vektor Informationen bezüglich Wechselbeziehungen der mehreren Aminosäuren, aus denen die Peptidsequenz des entsprechenden Eintrags der Peptidsequenz der Alpha-Kette besteht, umfasst,
∘ Verarbeiten des codierten numerischen Eingabevektors und des entsprechenden numerischen Ausgabe-Aufmerksamkeits-Vektors zu einem numerischen Korrelationsvektor unter Verwendung einer Teilschicht mit mehrfacher Encoder-Decoder Aufmerksamkeit (Multi-headed Encoder-Decoder Attention Sublayer) und einer Feed-Forward-Teilschicht, wobei der numerische Korrelationsvektor Korrelationsinformationen zwischen dem codierten numerischen Eingabevektor und dem entsprechenden Ausgabe-Aufmerksamkeits-Vektor umfasst, und
∘ Verarbeiten des numerischen Korrelationsvektors zu einer Wahrscheinlichkeit der Entsprechung zwischen dem eingebetteten numerischen Eingabevektor und dem eingebetteten numerischen Ausgabevektor unter Verwendung eines Wahrscheinlichkeitsgenerators.

9. Verfahren nach dem vorhergehenden Anspruch 8, wobei das Verarbeiten eines Paares der mehreren Eingabe-Ausgabe-Paare ferner den folgenden Schritt umfasst:
∘ Gewinnen eines Datenpunkts einer Score-Funktion zum Trainieren durch Vergleichen der Wahrscheinlichkeit einer Entsprechung zwischen dem eingebetteten numerischen Eingabevektor und dem eingebetteten numerischen Ausgabevektor mit entsprechenden Relationsinformationen, die mit dem Trainingsdatensatz assoziiert sind,
∘ Anpassen eines mit dem Deep-Learning-Modell assoziierten Parameters, um die Score-Funktion zu optimieren,
vorzugsweise wobei die Score-Funktion eines oder mehreres von einer Quadratsummenfehler-Score-Funktion, einer Mittelwert-Score-Funktion oder einer Maximum-Score-Funktion ist.

10. Verfahren nach einem der vorhergehenden Ansprüche 7 bis 9, wobei das Transformer-Netz einen Encoder und einen Decoder umfasst,
wobei der Encoder Folgendes umfasst:
∘ einen Neoepitop-Einbetter,
∘ einen Positions-Encoder,
∘ einen oder mehrere Sequenz-Encoder, die jeweils zwei Teilschichten umfassen:
i. eine Teilschicht mit mehrfacher Selbstaufmerksamkeit (Multi-headed Self-Attention),
ii. eine Feed-Forward-Teilschicht,
wobei der Decoder Folgendes umfasst:
∘ einen oder mehrere Sequenz-Decoder, die jeweils drei Teilschichten umfassen:
i. eine Teilschicht mit mehrfacher Selbstaufmerksamkeit,
ii. eine Teilschicht mit mehrfacher Encoder-Decoder-Aufmerksamkeit (Multi-headed Encoder-Decoder Attention),
iii. eine Feed-Forward-Teilschicht,
∘ einen HLA-Sequenz-Einbetter,
∘ einen Wahrscheinlichkeitsgenerator, der Folgendes umfasst:
i. einen linearen Projektor,
ii. eine Softmax-Schicht.

11. Verfahren nach einem der vorhergehenden Ansprüche 1 bis 10, ferner die folgenden Schritte umfassend:
- Trainieren eines semi-unabhängigen neuronalen Netzes an einem semi-unabhängigen Trainingsdatensatz, der mindestens den positiven Datensatz des Deep-Learning-Modells oder eine Variante davon und einen assoziierten Vorhersageverbesserungsparameter-Trainingsdatensatz umfasst, wobei sich der assoziierte Vorhersageverbesserungsparameter-Trainingsdatensatz auf einen oder mehrere biologische Parameter der RNA-Expression eines Gens, von dem das Neoepitop abgeleitet ist, der RNA-Expression mehrerer Gene in einer Krebsgewebeprobe, der Expression nichtkodierender RNA-Sequenzen, Informationen zu posttranslationaler Modifikation, Informationen zu RNA-Editing-Ereignissen, Immunfraktionen mehrerer Immunzellentypen, Klonalität einer Krebsgewebeprobe, Konfidenzwert mehrerer genom-verändernder Ereignisse, Peptid-MHC-Bindungsaffinität, Stabilität des Peptid-MHC-Komplexes, Peptidstabilität und/oder -umsatz, benachbarte Aminosäuren innerhalb der Neoepitop-Sequenz, Proteasom-Aktivität und Peptidverarbeitungsaktivität bezieht, vorzugsweise wobei der assoziierte Vorhersageverbesserungsparameter-Trainingsdatensatz sich mindestens auf benachbarte Aminosäuren innerhalb der Neoepitop-Sequenz bezieht,
- Bestimmen einer semi-unabhängigen Präsentationswahrscheinlichkeit für jedes des Satzes von Neoantigenen für die Peptidsequenz des HLA mittels des trainierten semi-unabhängigen neuronalen Netzes, und
- Kombinieren der bestimmten semi-unabhängigen Präsentationswahrscheinlichkeit und der mittels des trainierten Modells gewonnenen Präsentationswahrscheinlichkeit für jedes des Satzes von Neoantigenen, um eine Gesamtpräsentationswahrscheinlichkeit zu gewinnen,
vorzugsweise wobei das Kombinieren mittels eines trainierten einschichtigen neuronalen Netzes durchgeführt wird,
vorzugsweise wobei das semi-unabhängige neuronale Netz ein einschichtiges neuronales Netz ist.

12. Computersystem zum Bestimmen von Präsentationswahrscheinlichkeiten eines Satzes von Neoantigenen durch eine Tumorzelle eines Tumors eines Subjekts, wobei das Computersystem dazu konfiguriert ist, das computerimplementierte Verfahren nach einem der vorhergehenden Ansprüche 1 bis 11 durchzuführen.

13. Computerprogrammprodukt zum Bestimmen von Präsentationswahrscheinlichkeiten eines Satzes von Neoantigenen durch eine Tumorzelle eines Tumors eines Subjekts, wobei das Computerprogrammprodukt Anweisungen umfasst, die, wenn das Computerprogrammprodukt von einem Computer ausgeführt wird, den Computer veranlassen, das computerimplementierte Verfahren nach einem der vorhergehenden Ansprüche 1 bis 11 auszuführen.

14. Verwendung des computerimplementierten Verfahrens nach einem der vorhergehenden Ansprüche 1 bis 11 und/oder des Computersystems nach dem vorhergehenden Anspruch 12 und/oder des Computerprogrammprodukts nach dem vorhergehenden Anspruch 13, zum Bestimmen einer Behandlung für das Subjekt.

15. Verwendung nach dem vorhergehenden Anspruch 14, wobei das Bestimmen einer Behandlung Folgendes umfasst: Auswählen eines Teilsatzes des identifizierten Satzes von Neoantigenen basierend auf den bestimmten Präsentationswahrscheinlichkeiten, um einen Teilsatz ausgewählter Neoantigene zu gewinnen, vorzugsweise wobei der Teilsatz durch Vergleichen der Präsentationswahrscheinlichkeit jedes des Satzes von Neoantigenen mit einem Schwellenwert gewonnen wird, wobei ein Neoantigen dem Teilsatz hinzugefügt wird, wenn die assoziierte Präsentationswahrscheinlichkeit den Schwellenwert überschreitet, und Identifizieren einer oder mehrerer T-Zellen, die für mindestens eines der Neoantigene in dem Teilsatz antigen-spezifisch sind.

## Revendications

1. Procédé mis en oeuvre par ordinateur pour déterminer des probabilités de présentation d'un ensemble de néoantigènes par une cellule tumorale d'une tumeur d'un sujet, le procédé comprenant les étapes consistant à:
- obtenir au moins l'une parmi des données de séquençage de nucléotides de l'exome ou du génome entier et des données de séquençage de nucléotides du transcriptome à partir de cellules tumorales associées à ladite tumeur et de cellules normales du sujet;
- obtenir un ensemble d'événements génomiques aberrants associés à ladite tumeur en comparant les données de séquençage de nucléotides de l'exome et/ou du génome entier et les données de séquençage de nucléotides du transcriptome provenant des cellules tumorales aux données de séquençage de nucléotides de l'exome et/ou du génome entier et aux données de séquençage de nucléotides du transcriptome provenant des cellules normales;
- obtenir des données représentant des séquences peptidiques de chacun d'un ensemble de néoantigènes identifiés sur la base au moins en partie dudit ensemble d'événements aberrants, dans lequel la séquence peptidique de chaque néoantigène comprend au moins une altération qui la distingue d'une séquence peptidique de type sauvage correspondante identifiée à partir des cellules normales du sujet;
- obtenir des données représentant une séquence peptidique d'un antigène leucocytaire humain (HLA) sur la base des données de séquençage de nucléotides de l'exome et/ou du génome entier de la tumeur et des données de séquençage de nucléotides du transcriptome provenant des cellules tumorales;
- entraîner un modèle d'apprentissage profond sur un ensemble de données d'apprentissage comprenant un ensemble de données positives, dans lequel l'ensemble de données positives comprend une pluralité de paires d'entrée-sortie, dans lequel chaque paire comprend une entrée d'une séquence d'épitope en tant qu'entrée, ladite séquence d'épitope étant identifiée ou déduite d'un complexe HLA/peptide lié à la surface ou sécrété codé par un allèle HLA correspondant exprimé par une cellule d'apprentissage, dans lequel chaque paire comprend en outre une entrée d'une séquence peptidique d'une chaîne alpha codée par l'allèle HLA correspondant en tant que sortie; et
- déterminer une probabilité de présentation pour chacun de l'ensemble de néoantigènes pour la séquence peptidique du HLA au moyen du modèle entraîné.

2. Procédé selon la revendication précédente 1, comprenant en outre les étapes consistant à:
- associer un score de confiance à chacun dudit ensemble d'événements génomiques aberrants sur la base au moins en partie d'un nombre de lectures de séquençage des données de séquençage prenant en charge chaque événement génomique aberrant associé;
- obtenir un sous-ensemble d'événements génomiques aberrants en comparant le score de confiance de chaque événement génomique aberrant dudit ensemble d'événements génomiques aberrants à une valeur seuil, dans lequel un événement est ajouté audit sous-ensemble si le score de confiance associé dépasse ladite valeur seuil;
dans lequel ledit ensemble de néoantigènes est identifié sur la base au moins en partie dudit sous-ensemble d'événements génomiques aberrants.

3. Procédé selon l'une quelconque des revendications précédentes 1 ou 2, dans lequel l'ensemble de données positives comprend un ensemble de données monoalléliques et multialléliques, dans lequel l'ensemble de données monoalléliques comprend des paires d'entrée-sortie obtenues à partir d'une cellule d'apprentissage provenant d'une lignée cellulaire monoallélique et dans lequel l'ensemble de données multialléliques comprend des paires d'entrée-sortie obtenues à partir d'une cellule d'apprentissage provenant d'un tissu multiallélique.

4. Procédé selon la revendication précédente 3, dans lequel l'apprentissage du modèle d'apprentissage profond comprend deux cycles d'apprentissage ou plus, dans lequel chaque cycle d'apprentissage comprend une pluralité d'étapes d'apprentissage, dans lequel chaque étape d'apprentissage comprend le traitement d'une paire de la pluralité de paires d'entrée-sortie, dans lequel l'un desdits deux cycles d'apprentissage ou plus comprend l'apprentissage du modèle d'apprentissage profond sur l'ensemble de données monoalléliques et dans lequel l'un desdits deux cycles d'apprentissage ou plus comprend l'apprentissage du modèle d'apprentissage profond à la fois sur l'ensemble de données monoalléliques et sur l'ensemble de données multialléliques.

5. Procédé selon l'une quelconque des revendications précédentes 1 à 4, dans lequel l'ensemble de données d'apprentissage pour entraîner le modèle d'apprentissage profond comprend en outre un ensemble de données négatives comprenant une pluralité de paires d'entrée-sortie, chaque paire comprenant une entrée d'une séquence peptidique en tant qu'entrée, dans lequel ladite séquence peptidique est une séquence aléatoire d'un protéome humain et dans lequel chaque paire comprend en outre une séquence peptidique codée à partir d'un allèle HLA aléatoire en tant que sortie.

6. Procédé selon l'une quelconque des revendications précédentes 1 à 5, dans lequel le modèle d'apprentissage profond est au moins l'un parmi un modèle de similarité sémantique profonde, un modèle de similarité sémantique profonde convolutif, un modèle de similarité sémantique profonde récurrent, un modèle de correspondance de pertinence profonde, un modèle profond et large, un modèle de langage profond, un réseau transformateur, un réseau de longue mémoire à court terme, une intégration de texte d'apprentissage profond appris, une reconnaissance d'entité nommée apprise, un réseau neuronal siamois, un réseau siamois d'interaction ou un réseau de correspondance lexicale et sémantique, ou des combinaisons de ces modèles.

7. Procédé selon l'une quelconque des revendications précédentes 1 à 6, dans lequel le modèle d'apprentissage profond est un réseau transformateur.

8. Procédé selon l'une quelconque des revendications précédentes 1 à 7, dans lequel l'apprentissage du modèle d'apprentissage profond comprend une pluralité d'étapes d'apprentissage, chaque étape d'apprentissage comprenant le traitement d'une paire de la pluralité de paires d'entrée-sortie selon les étapes consistant à:
∘ traiter l'entrée de la paire en un vecteur numérique d'entrée intégré en convertissant l'entrée correspondante d'une séquence d'épitopes à l'aide d'un intégrateur de néoépitopes et d'un codeur positionnel, le vecteur numérique d'entrée intégré comprenant des informations relatives à une pluralité d'acides aminés qui composent la séquence d'épitopes de l'entrée correspondante et un ensemble de positions d'acides aminés dans la séquence d'épitopes;
∘ traiter la sortie de la paire en un vecteur numérique de sortie intégré en convertissant l'entrée correspondante de la séquence peptidique de la chaîne alpha à l'aide d'un intégrateur d'allèles et d'un codeur positionnel, le vecteur numérique de sortie intégré comprenant des informations relatives à la pluralité d'acides aminés qui composent la séquence peptidique de l'entrée correspondante et un ensemble de positions des acides aminés dans la séquence peptidique;
∘ traiter le vecteur numérique d'entrée intégré en un vecteur numérique d'entrée codé à l'aide d'au moins un codeur de séquence comprenant une sous-couche d'auto-attention à plusieurs têtes et une sous-couche de propagation avant, le vecteur numérique d'entrée codé comprenant des informations relatives à une caractéristique de la séquence d'épitopes de l'entrée correspondante de la séquence d'épitopes;
∘ traiter le vecteur numérique de sortie intégré en un vecteur numérique d'attention de sortie à l'aide d'une sous-couche d'auto-attention à plusieurs têtes, le vecteur numérique d'attention de sortie comprenant des informations relatives aux interdépendances de la pluralité d'acides aminés qui composent la séquence peptidique de l'entrée correspondante de la séquence peptidique de la chaîne alpha;
∘ traiter le vecteur numérique d'entrée codé et le vecteur d'attention de sortie correspondant en un vecteur numérique de corrélation à l'aide d'une sous-couche d'attention de codeur-décodeur à plusieurs têtes et d'une sous-couche de propagation avant, le vecteur numérique de corrélation comprenant des informations de corrélation entre le vecteur numérique d'entrée codé et le vecteur d'attention de sortie correspondant; et
∘ traiter le vecteur numérique de corrélation en une probabilité de correspondance entre le vecteur numérique d'entrée intégré et le vecteur numérique de sortie intégré à l'aide d'un générateur de probabilités.

9. Procédé selon la revendication précédente 8, dans lequel le traitement d'une paire de la pluralité de paires d'entrée-sortie comprend en outre l'étape consistant à:
∘ obtenir un point de données d'une fonction de score pour l'apprentissage en comparant la probabilité de correspondance entre le vecteur numérique d'entrée intégré et le vecteur numérique de sortie intégré à des informations de relation correspondantes associées à l'ensemble de données d'apprentissage;
∘ ajuster un paramètre associé au modèle d'apprentissage profond afin d'optimiser ladite fonction de score;
de préférence, dans lequel la fonction de score est une ou plusieurs parmi une fonction de score de la somme des erreurs quadratiques, une fonction de score moyen ou fonction de score maximal.

10. Procédé selon l'une quelconque des revendications précédentes 7 à 9, dans lequel le réseau transformateur comprend un codeur et un décodeur;
le codeur comprenant:
∘ un intégrateur de néoépitopes;
∘ un codeur positionnel;
∘ un ou plusieurs codeurs de séquence, chacun comprenant deux sous-couches:
i. une sous-couche d'auto-attention à plusieurs têtes;
ii. une sous-couche de propagation avant;
le décodeur comprenant:
∘ un ou plusieurs décodeurs de séquence, chacun comprenant trois sous-couches:
i. une sous-couche d'auto-attention à plusieurs têtes;
ii. une sous-couche d'attention de codeur-décodeur à plusieurs têtes;
iii. une sous-couche de propagation avant;
∘ un intégrateur de séquences HLA;
∘ un générateur de probabilités, comprenant;
i. un projecteur linéaire;
ii. une couche softmax.

11. Procédé selon l'une quelconque des revendications précédentes 1 à 10, comprenant en outre les étapes consistant à:
- entraîner un réseau neuronal semi-indépendant sur un ensemble de données d'apprentissage semi-indépendant comprenant au moins l'ensemble de données positives du modèle d'apprentissage profond ou une variante de celui-ci et un ensemble de données d'apprentissage de paramètres d'amélioration de prédiction associé, dans lequel ledit ensemble de données d'apprentissage de paramètres d'amélioration de prédiction associé se rapporte à un ou plusieurs paramètres biologiques de l'expression de l'ARN d'un gène dont le néoépitope est dérivé, de l'expression de l'ARN d'une pluralité de gènes dans un échantillon de tissu cancéreux, de l'expression de séquences d'ARN non codantes, d'informations sur les modifications post-traductionnelles, d'informations sur les événements d'édition de l'ARN, de fractions immunitaires d'une pluralité de types de cellules immunitaires, de la clonalité d'un échantillon de tissu cancéreux, d'un score de confiance d'une pluralité d'événements modifiant le génome, de l'affinité de liaison peptide-MHC, de la stabilité du complexe peptide-MHC, de la stabilité et/ou du renouvellement du peptide, des acides aminés voisins dans la séquence du néoépitope, de l'activité du protéasome, et de l'activité de traitement du peptide, de préférence dans lequel ledit ensemble de données d'apprentissage de paramètres d'amélioration de prédiction associé concerne au moins des acides aminés voisins dans la séquence du néoépitope;
- déterminer une probabilité de présentation semi-indépendante pour chacun de l'ensemble de néoantigènes pour la séquence peptidique de l'HLA au moyen du réseau neuronal semi-indépendant entraîné; et
- combiner, pour chacun de l'ensemble de néoantigènes, la probabilité de présentation semi-indépendante déterminée et la probabilité de présentation obtenue au moyen du modèle entraîné, afin d'obtenir une probabilité de présentation globale;
de préférence dans lequel la combinaison est effectuée au moyen d'un réseau neuronal entraîné à une seule couche;
de préférence dans lequel le réseau neuronal semi-indépendant est un réseau neuronal à une seule couche.

12. Système informatique permettant de déterminer des probabilités de présentation d'un ensemble de néoantigènes par une cellule tumorale d'une tumeur d'un sujet, le système informatique étant configuré pour exécuter le procédé mis en oeuvre par ordinateur selon l'une quelconque des revendications précédentes 1 à 11.

13. Produit-programme d'ordinateur pour déterminer des probabilités de présentation d'un ensemble de néoantigènes par une cellule tumorale d'une tumeur d'un sujet, le produit-programme d'ordinateur comprenant des instructions qui, lorsque le produit-programme d'ordinateur est exécuté par un ordinateur, amènent l'ordinateur à exécuter le procédé mis en oeuvre par ordinateur selon l'une quelconque des revendications précédentes 1 à 11.

14. Utilisation du procédé mis en oeuvre par ordinateur selon l'une quelconque des revendications précédentes 1 à 11 et/ou du système informatique selon la revendication précédente 12 et/ou du produit-programme d'ordinateur selon la revendication précédente 13, pour déterminer un traitement pour le sujet.

15. Utilisation selon la revendication précédente 14, dans laquelle la détermination d'un traitement comprend: la sélection d'un sous-ensemble de l'ensemble identifié de néoantigènes sur la base des probabilités de présentation déterminées afin d'obtenir un sous-ensemble de néoantigènes sélectionnés, de préférence dans lequel le sous-ensemble est obtenu en comparant la probabilité de présentation de chacun de l'ensemble de néoantigènes à une valeur seuil, dans lequel un néoantigène est ajouté audit sous-ensemble si la probabilité de présentation associée dépasse ladite valeur seuil; et l'identification d'une ou plusieurs cellules T qui sont spécifiques de l'antigène pour au moins l'un des néoantigènes dans ledit sous-ensemble.
